# EUROPÄISCHE PATENTSCHRIFT

(11) **EP 2 162 183 B1**
(45) Veröffentlichungstag und Bekanntmachung des Hinweises auf die Patenterteilung: **26.06.2019**
(21) Anmeldenummer: 08784267.0
(22) Anmeldetag: 25.06.2008
(51) Int. Cl.: A61N 1/32, A61K 9/70, A61K 31/4468, A61N 1/30, A61K 9/00

(54) **ELEKTROPHORETISCHES TRANSDERMALES APPLIKATIONSSYSTEM**
ELECTROPHORETIC TRANSDERMAL APPLICATION SYSTEM
SYSTÈME D'ADMINISTRATION TRANSDERMIQUE ÉLECTROPHORÉTIQUE

(30) Priorität: 25.06.2007 DE 102007029139
(43) Veröffentlichungstag der Anmeldung: 17.03.2010
(73) Patentinhaber: AMW GmbH, 83627 Warngau (DE)
(72) Erfinder: MEYER, Elisabeth, 83714 Miesbach (DE); SCHRÖDER, Britta, 82008 Unterhaching (DE); ZOGLMEIER, Christine, 83109 Grosskarolinenfeld (DE); NICKEL, Ulrich, 91058 Erlangen (DE)
(74) Vertreter: Beckord & Niedlich Patentanwälte PartG mbB
(86) Internationale Anmeldenummer: PCT/DE2008/001070
(87) Internationale Veröffentlichungsnummer: WO 2009/000262

(56) Entgegenhaltungen:
- WO-A-93/03790
- WO-A-95/06497
- WO-A-2007/038555
- US-A- 5 622 530
- US-B1- 6 757 560
- ROY S D ET AL: "Transdermal delivery of narcotic analgesics: comparative permeabilities of narcotic analgesics through human cadaver skin." Oktober 1989 (1989-10), PHARMACEUTICAL RESEARCH OCT 1989, VOL. 6, NR. 10, PAGE(S) 825 - 832 , XP002502872 ISSN: 0724-8741 das ganze Dokument

## Beschreibung

Die vorliegende Erfindung bezieht sich auf ein elektrophoretisches transdermales Applikationssystem (Transdermal Delivery System, TDS) für die Verabreichung zumindest eines Wirkstoffes durch die Haut.

Mit transdermalen therapeutischen Systemen werden Wirkstoffe durch die Haut hindurch in das Blutgefäßsystem eingebracht. Das Stratum corneum der Haut stellt eine natürliche Barriere dar, die von den Wirkstoffen überwunden werden muss. Für den Wirkstofftransport durch die Haut wird die passive Diffusion entlang des Wirkstoffkonzentrationsgefälles zwischen dem TDS und dem Stratum corneum der Haut ausgenutzt.

Die hautgängigen Wirkstoffe gelangen in der Regel ohne weitere Veränderungen in den Blutkreislauf. Die Freisetzung des Wirkstoffs aus dem TDS kann jedoch nur bedingt gesteuert werden. Mit der Freisetzung des Wirkstoffs nimmt dessen Konzentration in der hautnahen Schicht des TDS relativ schnell ab, sodass er nicht mit konstanter Abgaberate durch die Haut permeieren werden kann. Außerdem gelingt es durch eine solche passive konzentrationsabhängige Diffusion nicht, größere bzw. komplexere Wirkstoffmoleküle, wie beispielsweise Hormone durch die Haut zu treiben.

Den wichtigsten begrenzenden Faktor für ein transdermales therapeutisches System stellt die Durchlässigkeit des Stratum corneum für einen Wirkstoff dar. Um die gewünschte therapeutische Wirkung erzielen zu können, muss der Wirkstoff in ausreichender Menge durch die Haut hindurch in den Blutkreislauf gelangen können. Eine verbesserte Hautpermeation kann beispielsweise mithilfe der Iontophorese erreicht werden, die einen pulsatilen Transport der Wirkstoffe durch die Haut ermöglicht. Die Iontophorese basiert auf dem Transport von ionisierten Molekülen entlang eines die Haut durchdringenden elektrischen Feldes. Das Feld wird üblicherweise von zwei Elektroden erzeugt, die auf die Haut aufgebracht und von einer Stromquelle versorgt werden. Entlang des durch die Potentialdifferenz zwischen den Elektroden gebildeten Feldes werden die ionisch vorliegenden Wirkstoffmoleküle aus einem elektrisch leitfähigen Wirkstoffreservoir heraus und in die Haut hinein getrieben. Ein Vorteil der Iontophorese besteht insbesondere in der Möglichkeit, auch Makromoleküle und sogar Peptide wie Insulin partiell über die Haut verfügbar zu machen.

Der Aufbau von iontophoretischen Systemen erfordert grundsätzlich eine Kathode und eine Anode, um einen direkten Stromfluss durch den Körper zu erzeugen. Die Elektroden sind über wässrige, bisweilen in Gelen immobilisierte, Pufferlösungen verbunden, wodurch der Fluss der Wirkstoffionen durch die Haut sichergestellt wird. Damit jedoch kein Kurzschluss zwischen den Elektroden auftreten kann, müssen diese auf der Haut in ausreichendem Abstand zueinander aufgebracht werden, sodass iontophoretische Systeme groß ausfallen. Iontophoretische transdermale therapeutische Systeme sind bereits in einer Vielzahl von Druckschriften beschrieben worden, wobei beispielsweise auf US 5,622,530, WO 95/06497, US 6,757,560, US 3,991,755, US 4,141,359, DE 37 03 321, WO 87/04936, WO 91/16077, WO 92/04938, EP 0 532 451, US 5,415,629 und WO00/53256 verwiesen werden kann.

Bei elektrophoretischen transdermalen Systemen können ionische Wirkstoffe mit Hilfe von zwei Elektroden und einer Stromquelle durch die Haut getrieben werden, ohne dass der Körper einem direkten Stromfluss ausgesetzt ist. Bei diesen Systemen sind die Elektroden ähnlich einem Kondensator einander gegenüber angeordnet, wobei sich das Wirkstoffreservoir zwischen den Elektroden befindet und nur eine Elektrode hautseitig angeordnet ist.

In der Druckschrift US 5,533,995 ist ein solches elektrophoretisches transdermales Applikationssystem beschrieben worden. Es umfasst ein Reservoir, in dem der Wirkstoff enthalten ist, und ein Elektrodensystem, das so betrieben werden kann, dass der Wirkstoff in kontrollierter Weise aus dem Reservoir an die Haut abgegeben wird.

Ein haftklebendes transdermales Applikationssystem mit Elektrodenraster ist aus der Druckschrift EP 1 457 233 bekannt. Das System umfasst eine elektronische Steuerung der Elektrodenspannung, eine für den Wirkstoff undurchlässige Trägerfolie (Abdeckfolie), eine für den Wirkstoff durchlässige Membran, ein wirkstoffhaltiges Reservoir, das zwischen der Trägerfolie und der Membran angeordnet ist, und eine Abziehfolie. Die an der Trägerfolie angebrachte Elektrode dient als Gegenelektrode für die an der Membran angebrachte Elektrode. Das System umfasst ferner einen druckempfindlichen Haftkleber zur Befestigung des TDS auf der Hautoberfläche.

Die Aufgabe der vorliegenden Erfindung besteht darin, ein gegenüber dem Stand der Technik verbessertes elektrophoretisches transdermales System anzugeben.

Die vorliegende Erfindung bezieht sich daher auf ein transdermales Applikationssystem, das eine Matrix umfasst, die zumindest einen basischen oder zumindest einen sauren Wirkstoff enthält, sowie eine erste Elektrode und eine zweite Elektrode, die für den zumindest einen Wirkstoff durchlässig ausgebildet ist. Die Matrix des Applikationssystems ist zwischen der ersten Elektrode und der zweiten Elektrode angeordnet und weist einen pH-Wert auf, der im Falle eines basischen Wirkstoffes kleiner als der pKs-Wert des zumindest einen basischen Wirkstoffes ist und im Falle eines sauren Wirkstoffs größer als der pKs-Wert des zumindest einen sauren Wirkstoffes ist. Das System ist ferner so ausgelegt, dass durch eine an die Elektroden angelegte Spannung der pH-Wert der Matrix im Falle eines basischen Wirkstoffes nicht über den pKs-Wert des zumindest einen basischen Wirkstoffes steigt und im Falle eines sauren Wirkstoffes nicht unter den pKs-Wert des zumindest einen sauren Wirkstoffes fällt.

Die vorliegende Erfindung bezieht sich ferner auf ein Verfahren zur Verabreichung eines Wirkstoffes durch die Haut, bei dem ein derartiges transdermales Applikationssystem auf einen Hautbereich des menschlichen oder tierischen Körpers aufgebracht und für eine vorgegebene Applikationsdauer dort belassen wird, wobei zur Verabreichung des Wirkstoffes zwischen der ersten Elektrode und der zweiten Elektrode ein- oder mehrmals eine Spannung in der Weise angelegt wird, dass im Falle eines basischen Wirkstoffes der pH-Wert der Matrix nicht über den pKs-Wert des zumindest einen Wirkstoffes steigt und im Falle eines sauren Wirkstoffes der pH-Wert der Matrix nicht unter den pKs-Wert des zumindest einen Wirkstoffes fällt.

Die vorliegende Erfindung umfasst auch die Verwendung eines wie oben angegebenen transdermalen Applikationssystems zur Verabreichung zumindest eines Wirkstoffes durch die Haut.

Durch das erfindungsgemäße transdermale Applikationssystem ist es möglich, den oder die Wirkstoffe in einer solchen Menge freizusetzen, dass nach der Applikation des Systems am Patienten über einen längeren Zeitraum, wie z.B. 24 Stunden und insbesondere 72 Stunden oder länger vorgegebene Plasmaspiegel realisiert werden können.

Es können alle sauren oder basischen Wirkstoffe verwendet werden, die eine systemische Wirkung haben und die zur Permeation durch die Haut befähigt sind. Da der Wirkstoff in dem erfindungsgemäßen System nicht vollständig in Neutralform, sondern zumindest zu einem erheblichen Teil ionisch vorliegt, ist die Gefahr seiner Oxidation und/oder des Ausfallens seiner in Wasser schwer löslichen Neutralform geringer.

Erfindungsgemäß ist es ferner möglich, die Nebenwirkungen des verwendeten Wirkstoffes zu minimieren, weil die Wirkstoffabgabe bedarfsgemäß zeitlich begrenzt erfolgen kann und so der Wirkstoff nur dann in die Blutbahn abgegeben wird, wenn er auch benötigt wird.

Weitere vorteilhafte Ausführungsformen sind Gegenstand der Unteransprüche.

Vorteilhaft liegt der basische Wirkstoff des transdermalen Applikationssystems zu wenigstens 75 %, insbesondere wenigstens zu 90 % und vorzugsweise zu wenigstens 99 % in protonierter Form (bei saueren Wirkstoffen analog in deprotonierter Form) vor, um sicherzustellen, dass der überwiegende Anteil des Wirkstoffs in gut löslicher Form vorliegt und das transdermale Applikationssystem somit eine gute Lagerfähigkeit aufweist. Außerdem vereinfacht sich damit die pH-Wert-Steuerung.

In der Matrix ist zumindest ein Wirkstoff enthalten, der entweder protonierbar oder deprotonierbar ist, d.h. ein saurer oder ein basischer Wirkstoff, wobei es sich um basische Verbindungen oder basische Salze, saure Verbindungen oder saure Salze und gegebenenfalls um zwitterionische Verbindungen handeln kann.

Besonders geeignet für die verschiedensten Anwendungen des transdermalen Applikationssystems sind basische oder saure Wirkstoffe, die unter Analgetika, µ-Opioid-Rezeptor-Antagonisten, anästhesierenden Wirkstoffen, Parasympathomimetika, Parasympatholytika, Antiemetika, Emetika, Sympathomimetika, Hormonen, Antimigränemitteln, Antiallergika, Antikonvulsiva, Antidementiva, Antidepressiva, Betablockern und Analeptika und deren Gemischen ausgewählt sind.

Der zumindest eine basische Wirkstoff ist vorzugsweise unter Fentanyl, Buprenorphin, Physostigmin, Rivastigmin, Scopolamin, Granisetron, Zolmitriptan, Sumatriptan, Salbutamol oder deren Gemischen ausgewählt.

Zur Verbesserung des Wirkstoffflusses durch die Haut enthält die Matrix nach einer bevorzugten Ausführungsform einen oder mehrere Zusatzstoffe, die unter penetrationsfördernden und/oder permeationsfördernden Stoffen, Konservierungsmitteln, Antioxidantien, Feuchthaltemitteln, Elektrolyten, Verdickungsmitteln, Emulgatoren, Klebrigmachern, Weichmachern, Ansäuerungsmitteln, Alkalisierungsmitteln, Puffern und Füllstoffen und deren Gemischen ausgewählt sind.

Eine vorteilhafte Verminderung einer Gasbildung innerhalb des transdermalen Applikationssystems wird erreicht, indem zumindest die als Anode betriebene Elektrode eine Silberanode ist und in der Matrix Chlorid (Cl⁻) enthalten ist.

Zum unkomplizierten Anbringen auf die Haut weist das transdermale Applikationssystem ferner eine haftklebende Schicht auf, die dazu vorgesehen ist, bei der Applikation des Systems mit der Haut in Kontakt zu kommen. Verzögerungen in der Wirkstoffabgabe an die Haut können minimiert werden, wenn in der haftklebenden Schicht ebenfalls der zumindest eine basische oder der zumindest eine saure Wirkstoff in einer geeignet gewählten Konzentration enthalten ist.

An der hautabgewandten Seite weist das transdermale Applikationssystem vorteilhaft eine wirkstoffundurchlässige und vorzugsweise gasdurchlässige Deckfolie auf, damit keine unbeabsichtigte Wirkstofffreisetzung aus dem System erfolgen kann und gleichzeitig aber die durch die Elektrolyse im System gebildeten Gase entweichen können.

Um als autarkes System verwendet werden zu können, kann das transdermale Applikationssystem ferner eine Spannungsversorgung, die vorzugsweise eine Spannungsquelle und eine Steuerungseinrichtung umfasst, aufweisen. Eine hohe Betriebssicherheit kann durch Verwendung von einem oder mehreren Abstandshaltern und/oder einem oder mehreren Stützelementen erzielt werden, wobei eine oder mehrere druckempfindliche Haftschichten deren Fixierung im System verbessern können. Als Schutz gegenüber Verschmutzung und unbeabsichtigter Wirkstofffreisetzung kann das transdermale Applikationssystem ferner eine ablösbare Schutzfolie aufweisen.

Weitere Merkmale der Erfindung ergeben sich aus der vorliegenden Beschreibung von Ausführungsbeispielen in Verbindung mit den Ansprüchen und den Figuren. Die einzelnen Merkmale können bei einer Ausführungsform gemäß der Erfindung je für sich oder zu mehreren verwirklicht sein. Bei der nachfolgenden Erläuterung einiger Ausführungsbeispiele der Erfindung wird auf die beiliegenden Figuren Bezug genommen, von denen
- Fig. 1: einen Querschnitt durch ein erfindungsgemäßes elektrophoretisches transdermales Applikationssystem in einer schematisierten Darstellung zeigt.
- Fig.2: ein Diagramm des Wirkstoffflux durch die Haut für verschiedene transdermale Applikationssysteme zeigt,
- Fig.3: ein Diagramm der kumulierten Wirkstoffmengenabgabe durch die Haut für die transdermalen Applikationssysteme von Figur 2 zeigt,
- Fig. 4: ein Diagramm des Wirkstoffflux durch die Haut bei Anlegen unterschiedlicher Spannungen über unterschiedliche Zeitintervalle an Elektroden eines erfindungsgemäßen elektrophoretischen transdermalen Applikationssystems zeigt, und
- Fig. 5: ein Diagramm des Blutspiegelverlaufs von Fentanyl über der Zeit zeigt, das mit einem erfindungsgemäßen elektrophoretischen transdermalen Applikationssystem unter Anwendung von zwei Spannungszyklen verabreicht wurde.

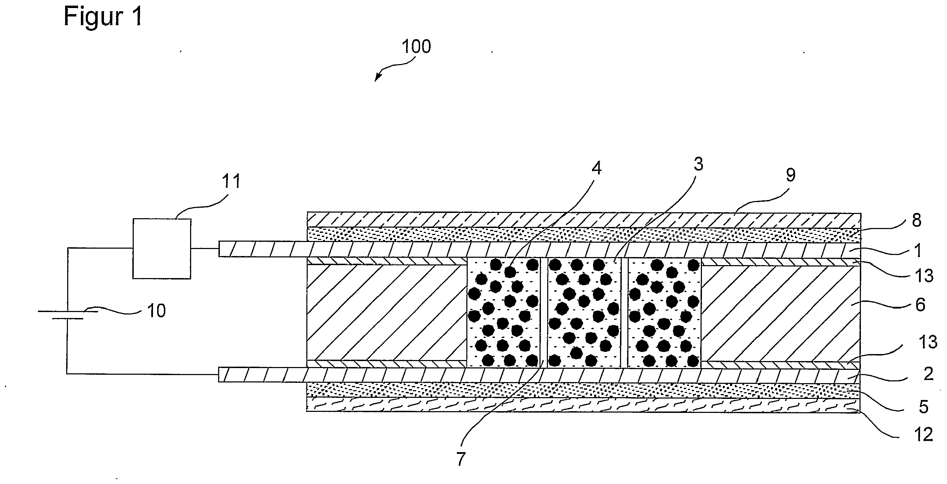

Das in der Fig. 1 dargestellte transdermale Applikationssystem 100 weist zwei Elektroden 1 und 2 auf, zwischen denen eine Matrix 3 angeordnet ist. Die Matrix 3 enthält den Wirkstoff 4. An der in der Figur unteren Elektrode 2 ist an deren der Matrix 3 abgewandten Seite eine Kleberschicht 5 zum Anbringen des transdermalen Applikationssystems auf einer Hautoberfläche vorgesehen. Die der Elektrode gegenüberliegende, freie Oberfläche der Kleberschicht 5 wird im Folgenden als hautseitige Oberfläche des transdermalen Applikationssystems 100 bezeichnet.

Die untere Elektrode 2 weist Öffnungen auf, durch die der Wirkstoff in die Kleberschicht 5 übertreten kann. Die gegenüberliegende Elektrode 1 ist ebenfalls mit Öffnungen versehen, kann aber auch mit einer geschlossenen Oberfläche ausgeführt sein. Die zwischen den Elektroden angeordneten Seitenflächen der Matrix 3 sind von einem Abstandshalter 6 eingefasst, der einem Austreten des Wirkstoffs bzw. der Matrix aus dem System entgegenwirkt und sicherstellt, dass sich die beiden Elektroden 1 und 2 nicht berühren. Überspannt die Matrix 3 einen größeren Bereich, so wird der Elektrodenabstand im Matrixbereich zweckmäßig mit einem oder mehreren Stützelementen 7 aufrechterhalten. Die der Matrix 3 abgewandte Seite der Elektrode 1 weist eine Kleberschicht 8 zur Befestigung der Deckfolie 9 auf. Die hautseitige Kleberschicht 5 kann zum Schutz vor Verschmutzung bei Nichtgebrauch mit einer ablösbaren Schutzfolie 12 bedeckt sein.

Elektrode 1 und Elektrode 2 sind jeweils so ausgebildet, dass sie mit einer Spannungsversorgung verbunden werden können. Wie in Figur 1 angedeutet, weist die Spannungsversorgung vorteilhaft eine zum Beispiel als Batterie ausgebildete Spannungsquelle 10 und eine Steuerungseinrichtung 11 auf, welche die Höhe und den zeitlichen Verlauf der zwischen den beiden Elektroden anliegenden Spannung steuert. Die Spannungsversorgung kann in das transdermale Applikationssystem 100 integriert sein, wobei die Steuerung 11 vorzugsweise als mikroelektronische Einheit, z.B. in Form eines integrierten Schaltkreises ausgebildet ist. Alternativ oder zusätzlich können die Elektroden oder die Spannungsversorgung auch zum Anschließen einer externen Spannungsversorgung ausgebildet sein.

Der beschriebene Aufbau des transdermalen Applikationssystems 100 dient dazu, einen oder mehrere der in der Matrix 3 enthaltenen Wirkstoffe 4 durch die hautseitige Elektrode 2 und die daran anschließende Kleberschicht 5 hindurch in ausreichender Konzentration kontrolliert über einen längeren Zeitraum hinweg zu der Hautoberfläche zu transportieren, auf der das System mithilfe der Kleberschicht 5 befestigt ist.

Im Prinzip ist es möglich, dass die Matrix als Flüssigkeit, in Gelform oder als selbsttragender Feststoff vorliegt. Der Fachmann wird natürlich im Falle einer flüssigen oder fließenden Matrix geeignete Vorkehrungen treffen, um ein "Ausfließen" der Matrix bzw. des oder der enthaltenden Wirkstoffe zu verhindern. Beispiele sind etwa das Vorsehen einer festen, flüssigkeitsundurchlässigen Umhüllung, die auf der der Haut zugewandten Seite von einer wirkstoffdurchlässigen Membran begrenzt wird, oder das Eindicken der Matrix mit Hilfe geeigneter Gelbildner. Vorzugsweise handelt es sich um eine Matrix auf Polymerbasis.

Für die Herstellung der Matrix eignen sich grundsätzlich alle Polymere, die bei der Herstellung von transdermalen Systemen eingesetzt werden und physiologisch unbedenklich sind, mit der Maßgabe, dass sie hydrophil oder gegebenenfalls amphiphil sind und entweder Wasser enthalten oder Wasser aufnehmen können. Besonders geeignet sind Hydrogele, beispielsweise synthetische Hydrogele, wie Poly(meth)acrylsäuren, Poly(meth)acrylate, Polyvinylpyrrolidon oder Polyvinylalkohol. Ebenso geeignet sind Hydrogele auf Cellulosebasis, wie z.B. Celluloseether. Insbesondere werden Hydroxyalkylcellulosen, wie beispielsweise Hydroxymethylcellulose, Hydroxyethylcellulose oder Hydroxypropylcellulose verwendet. Die Matrix kann gegebenenfalls haftklebend sein, alternativ kann auch eine (in der Figur 1 nicht gezeigte) Kleberschicht zwischen der Matrix 3 und der jeweils an sie angrenzenden Elektrode 1 bzw. 2 angeordnet sein.

Der Begriff "Matrix" ist, wie er in dieser Anmeldung verwendet wird, so zu verstehen, dass entweder die Matrix bereits Wasser enthält und als solche lagerfähig ist, oder durch Einbringen von Wasser kurz vor der Applikation des Systems 100 gegebenenfalls über die Haut Wasser aufnimmt.

In der Matrix 3 befindet sich ein bzw. befinden sich mehrere Wirkstoffe 4, wobei entweder basische oder saure Wirkstoffe verwendet werden können. Entsprechende Wirkstoffe zeichnen sich dadurch aus, dass sie in eine ionische Form überführt werden können. Im Folgenden wird ein transdermales Applikationssystem 100 unter Verwendung eines basischen Wirkstoffs beschrieben, wobei die beschriebene Funktionsweise analog auch für saure Wirkstoffe gilt. Die Wirkstoffe können in der Matrix in Form ihrer pharmazeutisch akzeptablen Salze enthalten sein.

In der Matrix des erfindungsgemäßen Systems 100 ist der pH-Wert so eingestellt, dass sein Wert kleiner als der pKs-Wert des Wirkstoffs ist, d.h. der basische Wirkstoff zu wenigstens 50% in protonierter Form als Kation vorliegt. In bevorzugten Ausführungsformen liegt der basische Wirkstoff zu wenigstens 75%, insbesondere wenigstens 90%, besonders bevorzugt zu wenigstens 99% und vorteilhaft zu wenigsten 99,9% in protonierter Form vor. Diese hohen Konzentrationen protonierten Wirkstoffes stellen sicher, dass der Wirkstoff in gelöster Form vorliegt. Denn die Neutralform des Wirkstoffes ist in Wasser schwer löslich und kann bei Überschreiten einer gewissen Konzentration aus der Lösung ausfallen, wodurch der Wirkstoff in der Matrix immobilisiert wird und somit nicht mehr frei verfügbar ist.

Beispiele für geeignete Wirkstoffe sind Analgetika, µ-Opioid-Rezeptor-Antagonisten, anästhesierende Wirkstoffe, Parasympathomimetika, Parasympatholytika, Antiemetika, Emetika, Sympathomimetika, Hormone, Antimigränemittel, Antiallergika, Antikonvulsiva, Antidementiva, Antidepressiva, Betablocker und Analeptika.

Bei den Analgetika kann es sich um Opioide handeln, wobei von diesen die reinen Agonisten, die gemischten Agonisten-Antagonisten, die Partialantagonisten und die reinen Antagonisten angegeben werden können. Reine Agonisten sind etwa Fentanyl, Remifentanil, Oxycodon und Methadon. Von den reinen Antagonisten können Naloxon und Naltrexon genannt werden. Ein gemischter Agonist-Antagonist ist z.B. Nalbuphin, ein Partialantagonist ist beispielsweise Buprenorphin. Salicylsäurederivate wie Acetylsalicylsäure, Etofenamat oder Diclofenac sind Beispiele für saure Analgetika.

Von den µ-Opioid-Rezeptor-Antagonisten können z.B. Almivopan und Methylnaltrexon genannt werden.

Von den anästhesierenden Wirkstoffen kommen etwa die Lokalanästhetika in Betracht, beispielsweise Lidocain, Tetracain oder Etidocain.

Beispiele für Parasympathomimetika sind die Cholinesterase-Hemmer, wobei von diesen insbesondere Physostigmin, Rivastigmin, Neostigmin, Donepezil und Galanthamin zu nennen sind.

Von den Parasympatholytika kann z.B. das Scopolamin angegeben werden.

Die Antiemetika können unter den Parasympatholytika, 5-HT₃-Rezeptor-Antagonisten, wie Ondansetron und Granisetron, und Dopamin-D₂-Rezeptor-Antagonisten, wie Domperidon, ausgewählt werden.

Von den Emetika kommen Dopamin-D₂-Rezeptor-Agonisten, wie A-pomorphin, in Betracht.

Von den Sympathomimetika kommen die Catecholamine in Betracht, wie Dobutamin. Ferner sind auch die β₂-Sympathomimetika zu nennen, wie Salbutamol, Fenoterol und Clenbuterol.

Die Hormone können z.B. unter Estradiol, Norelgestromin, Goserelin oder Buserelin ausgewählt sein.

Beispiele für Antimigräne-Mittel sind 5-HT₁-Rezeptor-Agonisten, wie Triptone und von diesen insbesondere Zolmitriptan, Sumatriptan oder Naratriptan.

Ein Beispiel für Antikonvulsiva ist das Gabapentin.

Als Antidementivum kann das Memantin genannt werden.

Von den Antihistaminika kommen die Antiallergika in Betracht, wie beispielsweise Mizolastin, Triprolidin und Desloratadin.

Von den Antidepressiva kann das Nortriptylin angegeben werden.

Ein geeigneter Betablocker ist etwa das Nebivolol.

Ein Beispiel für ein Analeptikum ist etwa das Methylphenidat.

Die Matrix kann auch mehr als einen Wirkstoff enthalten, etwa eine Kombination aus 2 Wirkstoffen, wie z.B. ein Parasympathomimetikum in Kombination mit einem Parasympatholytikum, insbesondere Physostigmin/Scopolamin, ein Analgetikum in Kombination mit einem Antiemetikum, wie Fentanyl in Kombination mit Granisetron, oder zwei Analgetika, wie einen µ-Rezeptor-Agonisten und einen µ-Rezeptor-Antagonisten, z.B. Fentanyl/Naloxon.

Zum Transport durch die Haut und die eventuelle Kleberschicht 5 sollte der Wirkstoff 4 in deprotonierter, d.h. in Neutralform vorliegen. Die Umwandlung eines Teils des in der Matrix enthaltenen protonierten Wirkstoffes 4 wird durch Anlegen einer Spannung an die Elektroden 1 und 2 erreicht. Hierdurch wird nicht nur ein die Matrix 3 durchdringendes elektrisches Feld erzeugt, das zu einer Wanderung der darin enthaltenen Ionen führt, sondern es werden vielmehr auch elektrochemische Vorgänge an der Elektrodenoberfläche ausgelöst.

Insbesondere reagieren die von der Kathode in das System injizierten Elektronen mit den vorliegenden Oxoniumionen (H₃O⁺) zu molekularem Wasserstoff und in unmittelbarer Nähe zur Kathode bleiben die Hydroxidionen (OH⁻) zurück. Die Hydroxidionen reagieren mit den Protonen am protonierten Wirkstoff, wodurch der Wirkstoff deprotoniert und in die Neutralform überführt wird. Durch die lokal erhöhte OH⁻-Konzentration kommt es zu einer Erhöhung des pH-Werts. Um zu verhindern, dass der deprotonierte Wirkstoff auskristallisiert und somit nicht zur Hautoberfläche wandern kann, sollte der pH-Wert an keiner Stelle innerhalb der Matrix 3 über den pKs-Wert des Wirkstoffs 4 ansteigen.

An der Anode geben die Hydroxidionen Elektronen ab, wodurch molekularer Sauerstoff gebildet wird und an der Elektrode Oxoniumionen zurückbleiben. Dabei entstehende Gasblasen können eine kontrollierte Anwendung des transdermalen Applikationssystems 100 beeinträchtigen. Um diesem vorzubeugen, können die als Anode fungierende obere Elektrode 1 und die sich darüber befindende Deckfolie 9 gasdurchlässig ausgebildet sein. Als Deckfolien 9 können beispielsweise Folien aus Polyestern, Polyolefinen wie Polyethylen oder Polypropylen, Polyvinylidenchlorid oder Polyurethanen mit Stärken von 5 bis 1000 µm verwendet werden, wie sie dem Fachmann bekannt sind. Als gasdurchlässige Elektroden eignen sich unter anderem gelochte, poröse und gewebeförmige Folienmaterialien.

Ferner kann die Bildung von Gasblasen stark vermindert werden, indem eine Silberanode in Verbindung mit einer hinreichenden Menge an Chlorid in der Matrix verwendet wird. In diesem Fall wird nämlich an der Anode kaum noch Sauerstoff gebildet, sondern in erster Linie Silber gemäß der Reaktion Ag + Cl⁻ → AgCl + e⁻ unter Bildung von Silberchlorid oxidiert, das sich an der Anodenoberfläche abscheidet. Unter Silberanode wird in diesem Zusammenhang eine Anode verstanden, die entweder aus Silber besteht oder zumindest an der Oberfläche, die der Matrix zugewandt ist, eine Silberbeschichtung aufweist. Als Silberanoden werden insbesondere silberbeschichtetes E-delstahlgewebe, aber auch gelochte Silberfolien oder silberbeschichtete Polyestergewebe bevorzugt.

Durch die bei Verwendung von Silberanoden verringerte Bildung von Oxoniumionen im Anodenraum steigt der pH-Wert auch in Anodennähe an, wodurch sich die Deprotonierung des Wirkstoffs über den gesamten Matrixbereich erstreckt. In diesem speziellen Fall ist es im Gegensatz zur Darstellung von Figur 1 daher auch möglich, die Anode als hautseitige Elektrode zu betreiben. Dies hätte zudem den Vorteil, dass der an der Kathode gebildete Wasserstoff durch die Kathode und die Deckfolie ausgasen kann, ohne durch die Matrix wandern zu müssen.

Vorzugsweise sind Kathode und Anode als gasdurchlässige Silberelektroden ausgeführt, d.h. aus Silber oder einem silberbeschichteten Material. Grundsätzlich kommen als Elektroden jedoch alle herkömmlichen Edelmetallelektroden infrage, wie z.B. aus Gold, Platin oder Palladium bestehende oder damit beschichtete Elektroden, als auch Edelstahl- oder Kupferelektroden bzw. kupferbeschichtete Elektroden. Weitere geeignete Elektroden sind solche auf Kohlenstoffbasis. Die Anode und die Kathode können natürlich auch aus unterschiedlichen Materialien ausgeführt sein.

Die Elektroden können wiederum als Gewebe, vorzugsweise als gitterförmiges Gewebe, oder als gelochte oder poröse Folien, bzw. als mit einem leitfähigen Material bedruckte bzw. strukturiert bedruckte Folien ausgeführt sein. Insbesondere kann die obere Elektrode 1 als leitfähiger strukturierter Aufdruck auf der Deckfolie ausgeführt sein. Bei Verwendung einer gitterförmigen oder gitterförmig bedruckten Elektrode kann die Maschenweite z.B. 0,001 bis 1 mm und insbesondere 0,01 bis 0,05 mm betragen.

Durch die Verwendung einer Kleberschicht 5 kann die Haftung des transdermalen Applikationssystems 100 auf der Hautoberfläche verbessert werden. Die Kleberschicht 5 ist natürlich so ausgebildet, dass eine Diffusion des Wirkstoffs 4 durch diese hindurch möglich ist.

Mögliche Haftkleber für die Kleberschicht 5 sind dem Fachmann bekannt, wobei insbesondere Homo- und Copolymere auf Acrylbasis, die vorzugsweise keine funktionelle Gruppen aufweisen, Natur- und Synthesekautschuke, Polyvinylacetat, Polyisobutylen und Kleber auf Siliconbasis zu nennen sind. Vorzugsweise handelt es sich um einen hydrophoben Haftkleber. Alternativ kann das transdermale Applikationssystem 100 auch mit einem zusätzlichen haftenden Pflaster zur Befestigung auf der Haut versehen werden, das gewöhnlich auch als Overtape bezeichnet wird.

Die Kleberschicht 5 kann im ursprünglichen Zustand, d.h. bevor eine Spannung an die Elektroden 1 und 2 angelegt wurde, wirkstofffrei sein. In diesem Fall muss der Wirkstoff nach Inbetriebnahme des Systems 100 zunächst durch die Kleberschicht hindurchwandern, bevor er die Hautoberfläche erreicht. Um diese Verzögerung der Wirkstoffabgabe an die Haut zu vermeiden, kann die Kleberschicht 5 bereits eine geeignete initiale Wirkstoffkonzentration aufweisen, die einen sofortigen oder zumindest früheren Beginn der Permeation des Wirkstoffs durch die Haut sicherstellt. Das Flächengewicht dieser Kleberschicht 5 kann beispielsweise im Bereich 10 bis 100 g/m² liegen.

Die Konzentration des Wirkstoffes 4 oder der Wirkstoffe 4 in der Matrix 3 und/oder der Kleberschicht 5 hängt von der Tragedauer des TDS, der jeweils gewünschten Freisetzungsrate und der Permeation des Wirkstoffs durch die Haut ab und kann in weiten Bereichen schwanken. Typische Konzentrationen liegen im Bereich von 0,1 bis 10 Gew.-% Wirkstoff 4, bezogen auf das Gesamtgewicht der Matrix 3.

Die Matrix 3 und/oder die Kleberschicht 5 können für transdermale Applikationssysteme übliche Zusatzstoffe enthalten, wie beispielsweise penetrationsfördernde und/oder permeationsfördernde Stoffe, Konservierungsmittel, Antioxidantien, Feuchthaltemittel, Elektrolyten, Verdickungsmittel, Emulgatoren, Klebrigmacher, Weichmacher, Ansäuerungsmittel, Alkalisierungsmittel und Füllstoffe und deren Gemische. Der Fachmann wird den oder die gegebenenfalls enthaltenen Zusatzstoff(e) und/oder deren Mengenanteile natürlich so auswählen, dass die vorteilhaften Eigenschaften des elektrophoretischen transdermalen Systems nicht oder nicht wesentlich beeinträchtigt werden.

Die ablösbare Schutzfolie 12 schützt in üblicher Weise das transdermale System vor Verschmutzung und verhindert einen vorzeitigen Austritt des Wirkstoffs. Es können z.B. siliconisierte Polyethylenterephthalatfolien oder mit Fluorpolymeren beschichtete Polyesterfolien verwendet werden.

Der bzw. die Abstandshalter 6 werden vorzugsweise aus einem schaumförmigen Material gefertigt. Bei Verwendung von nicht haftklebenden Materialien können die mit den Elektroden in Kontakt stehenden Oberflächen der Abstandshalter 6 mit einer Kleberschicht 13 versehen sein. Als Stützelement bzw. Stützelemente 7 dienen in der Regel Teflonringe.

Das unter Bezugnahme auf die Fig. 1 erläuterte Applikationssystem 100 ermöglicht es, mittels Elektrolyse den pH-Wert zumindest im Kathodenraum hinreichend hoch einzustellen, damit genügend ungeladener Wirkstoff freigesetzt wird, der in der Folge durch die Matrix und die gegebenenfalls vorhandene Kleberschicht 5, in die Haut und durch diese hindurch diffundieren kann, andererseits aber nicht so hoch, dass der ungeladene Wirkstoff kristallisiert und aufgrund der damit verbundenen Immobilisierung nicht mehr abgegeben werden kann.

Ein Vorteil des Systems ist darin zu sehen, dass die wirksame Konzentration des Wirkstoffes im Blut auf Verlangen sehr schnell erreicht und gegebenenfalls auch sehr schnell weiter erhöht werden kann, wobei unter schnell Zeitspannen wie unter 120 min, vorzugsweise unter 60 min und insbesondere unter 30 min zu verstehen sind. Hierdurch kann der Blutspiegel des Wirkstoffs einem sich ändernden Bedarf in einfacher Weise angepasst werden.

Der Wirkstofffluss durch die Haut ist über die Menge des in die Neutralform überführten Wirkstoffes sehr gut steuerbar, sodass die gewünschte Wirkstoffkonzentration im Blut über lange Zeiträume, wie beispielsweise zwei, drei oder mehr Tage, aufrechterhalten werden kann.

Ferner kann die im TDS enthaltene Wirkstoffmenge besser ausgenutzt werden, da im Vergleich mit herkömmlichen Systemen insgesamt mehr Wirkstoff aus dem System entnommen werden kann. Dies stellt sowohl im Hinblick auf die erforderliche Menge der meistens sehr teuren Wirkstoffe, als auch im Hinblick auf die gegebenenfalls erforderliche Entsorgung der gebrauchten Applikationssysteme einen zusätzlichen Vorteil dar.

Durch die gezielte Freisetzung des Wirkstoffs mittels einer geeigneten Steuerung der Elektrodenspannung kann die Permeation des Wirkstoffs präzise gesteuert werden, wodurch der Blutspiegel des Wirkstoffs innerhalb des gewünschten therapeutischen Fensters eingestellt und auch über lange Zeiträume aufrechterhalten werden kann.

Der anfängliche pH-Wert des Systems, d.h. der pH-Wert vor dem ersten Anlegen einer Spannung an die Elektroden 1 und 2, kann beispielsweise durch Ansäuern der Matrix mit einer physiologisch akzeptablen, anorganischen oder organischen Säure oder deren Gemischen auf den gewünschten Wert unterhalb des pKs-Werts des verwendeten Wirkstoffs eingestellt werden. Als Beispiele für geeignete Säuren sind Salzsäure, Phosphorsäure, Citronensäure, Sorbinsäure, Essigsäure zu nennen.

Nach einer vorteilhaften Ausführungsform kann die Matrix 3 auch einen herkömmlichen Puffer enthalten, da hierdurch die pH-Wert-Erhöhung besser gesteuert werden kann; es kann sich z.B. um einen Essigsäure/Acetat-Puffer oder einen Citronensäure/Citrat-Puffer handeln.

Bei Verwendung eines Wirkstoffes mit einem pKs-Wert in der Gegend von 9, z.B. Fentanyl (pKs = 8,4) oder Granisetron (pKs = 9,4), liegt der anfängliche pH-Wert vorzugsweise im Bereich von 2 bis 7, besonders bevorzugt im Bereich von 3 bis 6 und insbesondere in der Gegend von 4.

Durch Anlegen einer Spannung an die beiden Elektroden 1 und 2 steigt der pH-Wert wie oben beschrieben an der Kathode an, wodurch der Wirkstoff deprotoniert werden kann.

Die Spannung muss ausreichend hoch sein, damit die Wasserelektrolyse stattfinden kann. Der Schwellwert für die angelegte Spannung hängt natürlich sowohl von dem verwendeten Elektrodenmaterial als auch von dem verwendeten Elektrolyten (der Matrix) ab. Die jeweils geeignete Spannung kann der Fachmann beispielsweise über die auftretende Gasbildung, den messbaren Stromfluss und/oder mit einem geeignet gewählten pH-Indikator ermitteln. Es hat sich herausgestellt, dass Spannungen ab etwa 2 V für die meisten Systeme gut geeignet sind.

Damit der pH-Wert nicht über den pKs-Wert des verwendeten Wirkstoffes steigt, darf die zugeführte Ladungsmenge allerdings auch nicht zu groß sein, d.h. der eingebrachte Strom und/oder die Dauer der Stromzufuhr müssen entsprechend begrenzt werden.

Nach einer besonders bevorzugten Ausführungsform der Erfindung erreicht man die kontrollierte Erhöhung des pH-Werts im Kathodenraum durch wiederholtes Anlegen einer vorgegebenen Spannung für einen vorgegebenen Zeitraum. Die verwendete Spannung, die Zeitdauer des Anlegens der Spannung und die Abstände der Wiederholungen sind natürlich wiederum von dem verwendeten konkreten Applikationssystem und insbesondere dem enthaltenen Wirkstoff abhängig. Mögliche Größenordungen sind etwa: Spannung: 2-6 V über ein Zeitintervall von 10-60 s bei einer Wiederholrate von 12-24 h. Die Zeitintervalle, während deren die Elektrodenspannung anliegt, können natürlich auch kürzer gewählt werden, wobei das Anlegen der Elektrodenspannung dann in der Regel in kürzeren Zeitabständen wiederholt wird. Beispielsweise kann die Elektrodenspannung über Zeitintervalle von 2 bis 10 s angelegt werden, die sich in Abständen von 1 bis 15 Stunden wiederholen. Über das Verhältnis von Zeitintervall zu Wiederholrate kann die Wirkstoffpermeation in einfacher Weise an das gewünschte therapeutische Fenster angepasst werden.

Bei Verwendung einer modulierten und insbesondere pulsweitenmodulierten Elektrodenspannung kann die Änderung des pH-Werts innerhalb eines Zeitintervalls präzise gesteuert werden, da sich der E-lektronenfluss zwischen Elektroden und Matrix über die Modulation, beispielsweise über das Tastverhältnis, in weitem Umfang unabhängig von der Elektrodenspannung steuern lässt. Die Modulation erfolgt vorzugsweise mit Frequenzen im Bereich von wenigen Hertz bis einigen kHz.

Der pH-Wert kann mit Hilfe eines in die Matrix eingebrachten pH-Indikators überprüft und/oder überwacht werden, wobei der Umschlagpunkt des Indiktors vorzugsweise auf den gewünschten pH-Wert unterhalb des pKs-Werts des Wirkstoffs abgestimmt ist. Es sind alle pH-Indikatoren geeignet, die toxikologisch unbedenklich sind und die vorteilhaften Eigenschaften des Applikationssystems nicht beeinträchtigen. Beispiele für geeignete pH-Indikatoren sind Phenolphthalein, Bromthymolblau, Phenolrot und dergleichen.

Im Folgenden wird ein Beispiel für die Herstellung eines oben beschriebenen elektrophoretischen transdermalen Applikationssystems 100 angegeben.

Auf einen physikalisch vernetzten, geschlossenzelligen Polyolefinschaumstoff mit einer Dicke von etwa 1mm wird beidseitig eine druckempfindliche Haftschicht 13 aufgebracht. Aus dem Schaumstoff wird ein Ring mit einem Außendurchmesser von 20 mm und einer inneren Öffnung von 1 cm² ausgestanzt, der als Abstandshalter 6 für die Elektroden dient.

Als Matrix 3 dient ein Hydrogel, das in die Öffnung eingebracht wird. Das Hydrogel wird aus Wasser und etwa 2,5 Gew.-% Hydroxyethylcellulose gebildet, als Wirkstoff 4 wird Fentanylcitrat in das Hydrogel eingearbeitet. Der pH-Wert der Matrix 3 wird auf etwa 4 eingestellt. Die Matrix 3 wird auf beiden Seiten mit jeweils einer Elektrode 1 bzw. 2 von 16 mm Durchmesser versehen, wobei sowohl für die Anode 1 als auch die Kathode 2 ein silberbeschichtetes Edelstahlquadratmaschengewebe mit einer Maschenweite von 0,06 mm verwendet wird. Jede der Elektroden weist einen Anschlussstreifen auf, an den eine externe Spannungsversorgung 10, 11 angeschlossen werden kann. Auf die der Haut abgewandten Seite wird eine durchsichtige, einseitig mit einem selbstklebenden Haftkleber beschichtete Deckfolie 9 aufgebracht. Auf die untere, der Deckfolie 9 abgewandten Seite wird eine mit einem druckempfindlichen, hydrophoben Siliconkleber beschichtete Schutzfolie 12 aufgebracht. Die Schutzfolie 12 wird vor dem Aufkleben des TDS auf die Haut natürlich entfernt.

Selbstverständlich stellen die angegebenen Formen, Maße, Wirkstoffe, Zusatzstoffe und Konzentrationsangaben nur ein spezielles Beispiel für ein erfindungsgemäßes TDS 100 dar. Je nach Anwendungsart weist jedes erfindungsgemäßes TDS 100 natürlich andere Formen, Maße, Wirkstoffe, Zusatzstoffe oder Konzentrationen auf, die der Fachmann leicht anpassen kann.

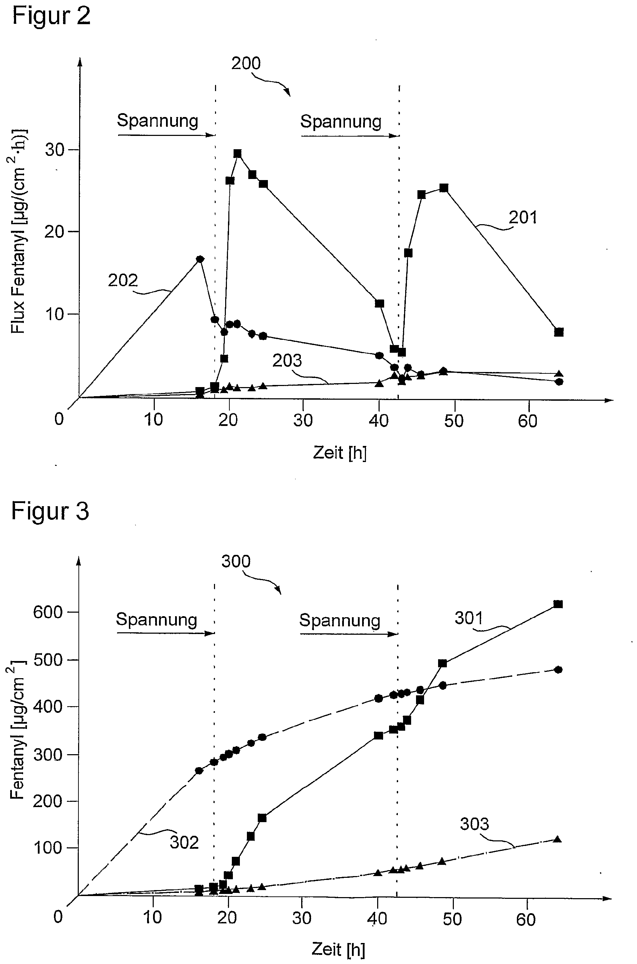

Das Diagramm 200 der Figur 2 zeigt den in einem *in vitro* Hautpermeationsversuch über der Zeit gemessenen Wirkstoffflux für verschiedene transdermale Applikationssysteme. Die angegebenen Werte stellen Mittelwerte aus n Messungen dar. Die Kurve 201 (n = 3) gibt die permeierte Rate des Wirkstoffes (Flux) Fentanyl aus einem wie oben beschriebenen elektrophoretischen TDS wieder, wobei 18,5 Stunden nach Versuchsbeginn erstmals eine Spannung von 2 V über einen Zeitraum von 60 Sekunden an die Elektroden des transdermalen Applikationssystems 100 angelegt wurde. 42,5 Stunden nach Versuchsbeginn wurde nochmals über einen gleichlangen Zeitraum eine Spannung von 3 V angelegt. Die Kurve 202 (n = 3) zeigt den mit einem im Handel von der Firma Janssen-Cilag erhältlichen, Fentanylhaltigen transdermalen Matrixsystem "Durogesic® SMAT 25 µg/h" erzielten Permeationsfluss, wobei die Wirkstoffabgabe passiv, d.h. ohne Anlegen einer Elektrodenspannung, erfolgt. Die Kurve 203 (n = 2) gibt den mit einem erfindungsgemäßen TDS erzielten Wirkstofffluss ohne Anlegen einer Elektrodenspannung wieder.

Die Wirkstoffabgabe des passiven Matrixsystems "Durogesic® SMAT 25 µg/h" nimmt über die ersten 16 Stunden des Versuchs zunächst zu, um im Anschluss daran in grober Näherung exponentiell abzufallen. Im Vergleich zum Matrixsystem "Durogesic® SMAT 25 µg/h" nimmt die Wirkstoffabgabe des elektrophoretischen transdermalen Applikationssystems 100 nur zögerlich zu und erreicht auch nach 17 Stunden keinen nennenswerten Flux. Mit dem Anlegen einer Elektrodenspannung (Kurve 201) etwa 18 Stunden nach Versuchsbeginn nimmt die Permeationsrate jedoch in kurzer Zeit stark zu und erreicht schließlich nahezu das Doppelte des von dem passiven Matrixsystem "Durogesic® SMAT 25 µg/h" erzielten Maximalwerts. Die Zunahme der Permeationsrate erstreckt sich über einen Zeitraum von circa 2 Stunden, also über eine wesentliche größere Zeitspanne als die, während der an den Elektroden 1 und 2 eine Spannung anlag. Nach Überschreiten der maximalen Permeationsrate nimmt die Wirkstoffabgabe im Vergleich zu "Durogesic® SMAT 25 µg/h" deutlich langsamer, in grober Näherung linear ab. Erst nach mehr als 20 Stunden entspricht der gemessene Fentanyl-Fluss in etwa dem von "Durogesic® SMAT 25 µg/h", was auf eine Erschöpfung des im transdermalen Applikationssystem 100 vorhanden Wirkstoffes in Neutralform hinweist. Eine circa 42 Stunden nach Versuchsbeginn erneut angelegte Elektrodenspannung von 3 V über einen Zeitraum von 60 Sekunden bewirkt innerhalb kurzer Zeit einen erneuten Anstieg der Wirkstoffabgabe, wobei der hierbei erzielte maximale Permeationsfluss etwas geringer als nach dem ersten Spannungsintervall ausfällt. Im Anschluss daran ist wieder ein in etwa linearer Abfall der Wirkstoffabgabe zu verzeichnen.

Im Gegensatz zum spannungsgesteuerten Betrieb des elektrophoretischen Systems 100 nimmt dessen Permeationsrate ohne Spannungssteuerung (Kurve 203) nur langsam und nur auf vergleichsweise geringe Werte (in etwa 10 Prozent des nach dem Anlegen einer Elektrodenspannung erreichten Maximalwerts) zu.

Im Vergleich zu einem kommerziell verfügbaren passiven System (siehe Kurve 202) können mit einem spannungsgesteuerten elektrophoretischen System somit höhere Permeationsraten über einen längeren Zeitraum erreicht werden, wobei die gewünschte Permeationsrate mittels der Parameter Elektrodenspannung, Dauer der Elektrodenspannung und Wiederholrate des Anlegens der Elektrodenspannung eingestellt werden kann. Daraus geht hervor, dass über diese Parameter der Wirkstoffspiegel im Blut eines Anwenders des TDS in weitem Umfang innerhalb eines erforderlichen Bereichs gehalten werden kann, wobei dieser Wirkstoffspiegel im Vergleich zu passiven Systemen über einen wesentlich längeren Zeitraum aufrechterhalten werden kann und sehr viel schneller erreicht wird.

Darüber hinaus ist die Gesamtmenge des pro Flächeneinheit aus einem elektrophoretischen transdermalen Applikationssystem 100 an die Haut abgegebenen Wirkstoffes wesentlich größer als bei passiven Systemen. Dies geht z.B. aus dem Diagramm 300 von Figur 3 hervor, in dem die Wirkstoffmengen aufgetragen sind, die über die Zeit von den in Fig. 2 gezeigten Systemen abgegeben wurden. Es handelt sich um eine kumulative Darstellung, d.h. jeder der Kurvenwerte gibt die von dem jeweiligen transdermalen Applikationssystem bis zu diesem Zeitpunkt abgegebene Wirkstoffmenge an.

Während die insgesamt von dem passiven System "Durogesic® SMAT 25 µg/h" abgegebene Wirkstoffmenge (Kurve 302) nach dem Überschreiten der maximalen Permeationsrate zunehmend abflacht und nach ca. 65 Stunden eine akkumulierte Menge von weniger als 500 µg/cm² erreicht, nimmt die Menge des von dem elektrophoretischen System 100 abgegebenen Wirkstoffs (Kurve 301) bereits kurz nach dem Anlegen der Elektrodenspannung sehr stark zu und erreicht schon ca. 40 Stunden nach dem ersten Anlegen einer Elektrodenspannung eine Gesamtabgabemenge von etwa 600 µg/cm². Ohne Anlegen einer Elektrodenspannung (Kurve 303) nimmt die Menge des vom elektrophoretischen System 100 abgegebenen Wirkstoffs jedoch nur geringfügig zu und erreicht auch nach ungefähr 65 Stunden nur eine Gesamtabgabemenge von in etwa 100 µg/cm².

Die vorgestellten Ergebnisse verdeutlichen, dass mit einem elektrophoretischen transdermalen Applikationssystem 100 durch wiederholtes Anlegen einer Elektrodenspannung für kurze Zeitintervalle eine schnelle Wirkstoffabgabe mit hohen Permeationsraten über einen langen Zeitraum möglich ist.

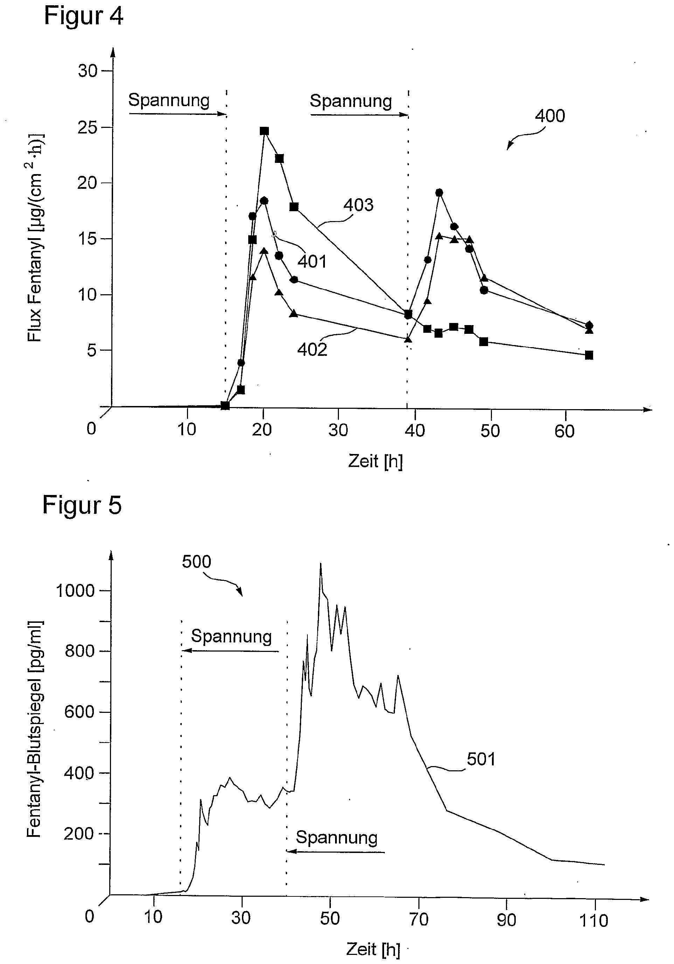

In Diagramm 400 der Figur 4 sind die mit drei gleichen Proben eines elektrophoretischen transdermalen Applikationssystems 100 bewirkten Permeationsflüsse des Wirkstoffs Fentanyl dargestellt, wobei sich die an die jeweiligen Proben angelegten Elektrodenspannungen in ihrer Höhe und Dauer voneinander unterscheiden. Die angegebenen Werte stellen Mittelwerte aus n Messungen dar. Bei allen drei Proben wurde zu denselben Zeitpunkten nach Versuchsbeginn zweimal eine Elektrodenspannung angelegt. Vor dem ersten Anlegen der Elektrodenspannungen ist der Permeationsfluss bei allen drei Proben äußerst gering und nimmt erst mit dem ersten Anlegen der Elektrodenspannung und in etwa gleich schnell zu. Allerdings unterscheiden sich die bei den verschiedenen Proben erzielten maximalen Permeationsflüsse sehr wesentlich.

Bei Anlegen einer Elektrodenspannung von 2V über einen Zeitraum von 60 Sekunden (Kurve 401, n = 2) wird eine maximale Permeationsrate von etwa 19 µg/(cm²·h) erreicht. Wird eine Elektrodenspannung von 3V über 15 Sekunden angelegt (Kurve 402, n = 3), so beträgt die maximale Permeationsrate weniger etwa 15 µg/(cm²·h). Der höchste maximale Flux von etwa 25 µg/(cm²·h) wird mit einer Elektrodenspannung von 9V über 60 Sekunden erzielt (Kurve 403, n= 3). Allerdings fällt der Flux in der Folge wesentlich stärker ab als bei den beiden anderen Proben.

Etwa 20 Stunden nach dem erstmaligen Anlegen einer Elektrodenspannung wird nochmals Spannung an die Elektroden angelegt. Während die maximalen Permeationsflüsse für die erste Probe (Kurve 401) und die zweite Probe (Kurve 402) in erster Näherung reprodoziert werden, ist bei der dritten Probe (Kurve 403) kein erneuter Anstieg des Permeationsflusses zu verzeichnen. Eine mögliche Erklärung hierfür ist, dass der deprotonierte Wirkstoff auskristallisiert, d.h. immobil geworden ist.

Im Diagramm 500 der Fig. 5 sind die Ergebnisse eines klinischen Versuchs veranschaulicht, bei dem einem Probanden ein elektrophoretisches transdermales Applikationssystem 100, wie oben beschrieben, appliziert wurde. Im Diagramm 500 ist der Fentanyl-Blutspiegel des Probanden über der Anwendungszeit als Kurve 501 aufgetragen. 16 Stunden und 40 Stunden nach dem Aufkleben des elektrophoretischen Systems wurde an die Elektroden 60 Sekunden lang eine Gleichspannung von 2,5 Volt angelegt. Die Fentanyl-Blutspiegel des Probanden wurden in regelmäßigen Abständen jede Stunde und unmittelbar nach dem Anlegen der Spannung im halbstündigen Abstand überprüft. Nach 65 Stunden wurden die Werte des Blutspiegels in zeitlich größeren Abständen aufgenommen.

Aus dem Diagramm ist ersichtlich, dass durch das Anlegen einer Spannung der Wirkstoff bereits nach etwa 2 Stunden durch die wirkstofffreie Kleberschicht 5 und die Haut in den Blutkreislauf gelangt ist. Der Blutspiegel des Wirkstoffs steigt schnell an und bleibt dann im Wesentlichen konstant. Durch das erneute Anlegen einer Spannung steigt die Konzentration des Fentanyls innerhalb kurzer Zeit im Blut nochmals stark an, bewegt sich ca. 15 Stunden auf hohem Niveau und fällt dann erst langsam ab. Nach 64 Stunden wurde das Pflaster entfernt, wobei der Blutspiegel etwa 3 Tage nach dem ersten Anlegen einer Elektrodenspannung noch immer bei Werten von über 200 pg/ml Fentanyl liegt.

Aus diesem Versuch wird deutlich, dass sich mit einem erfindungsgemäßen elektrophoretischen transdermalen Applikationssystem ein gewünschter Wirkstoff-Blutspiegel nicht nur schnell einstellen, sondern bei Bedarf auch noch weiter erhöhen lässt.

## Patentansprüche

1. Transdermales Applikationssystem (100) umfassend:
• eine Matrix (3), die zumindest einen basischen Wirkstoff (4) enthält,
• eine erste Elektrode (1), und
• eine zweite Elektrode (2), die für den zumindest einen Wirkstoff (4) durchlässig ausgebildet ist,
wobei die Matrix (3) zwischen der ersten Elektrode (1) und der zweiten Elektrode (2) angeordnet ist und einen pH-Wert aufweist, der kleiner als der pKs-Wert des zumindest einen Wirkstoffes (4) ist, und wobei das System so ausgelegt ist, dass durch eine an die Elektroden angelegte Spannung der pH-Wert der Matrix (3) nicht über den pKs-Wert des zumindest einen Wirkstoffes (4) steigt, wobei der basische Wirkstoff (4) zu wenigstens 75 % in protonierter Form vorliegt.

2. Transdermales Applikationssystem (100) nach Anspruch 1, worin der basische Wirkstoff (4) zu wenigstens 90 % und vorteilhaft zu wenigsten 99 % in protonierter Form vorliegt.

3. Transdermales Applikationssystem (100) nach Anspruch 1 oder 2, worin der zumindest eine basische Wirkstoff (4) unter Analgetika, µ-Opioid-Rezeptor-Antagonisten, anästhesierenden Wirkstoffen, Parasympathomimetika, Parasympatholytika, Antiemetika, Emetika, Sympathomimetika, Hormonen, Antimigränemitteln, Antiallergika, Antikonvulsiva, Antidementiva, Antidepressiva, Betablockern und Analeptika und deren Gemischen ausgewählt ist.

4. Transdermales Applikationssystem (100) nach Anspruch 3, worin der zumindest eine basische Wirkstoff (4) unter Fentanyl, Buprenorphin, Physostigmin, Rivastigmin, Scopolamin, Granisetron, Zolmitriptan, Sumatriptan, Salbutamol oder deren Gemischen ausgewählt ist.

5. Transdermales Applikationssystem (100) nach einem der vorher gehenden Ansprüche, worin die Matrix (3) einen oder mehrere Zusatzstoffe enthält, die unter penetrationsfördernden und/oder permeationsfördernden Stoffen, Konservierungsmitteln, Antioxidantien, Feuchthaltemitteln, Elektrolyten, Verdickungsmitteln, Emulgatoren, Klebrigmachern, Weichmachern, Ansteuerungsmitteln, Alkalisierungsmitteln, Puffern und Füllstoffen und deren Gemischen ausgewählt sind.

6. Transdermales Applikationssystem (100) nach einem der vorhergehenden Ansprüche, worin zumindest die als Anode betriebene Elektrode (1) eine Silberanode ist und in der Matrix (3) Chlorid (Cl⁻) enthalten ist.

7. Transdermales Applikationssystem (100) nach einem der vorhergehenden Ansprüche, das ferner eine haftklebende Schicht (5) aufweist, die dazu vorgesehen ist, bei der Applikation des Systems mit der Haut in Kontakt zu kommen.

8. Transdermales Applikationssystem (100) nach Anspruch 7, worin in der haftklebenden Schicht (5) ebenfalls der zumindest eine basische Wirkstoff (4) enthalten ist.

9. Transdermales Applikationssystem (100) nach einem der vorhergehenden Ansprüche, das ferner eine wirkstoffundurchlässige Deckfolie (9) umfasst, die vorzugsweise gasdurchlässig ausgebildet ist.

10. Transdermales Applikationssystem (100) nach einem der vorhergehenden Ansprüche, das ferner eine Spannungsversorgung, die vorzugsweise eine Spannungsquelle (10) und eine Steuerungseinrichtung (11) umfasst, und/oder einen oder mehrere Abstandshalter (6) und/oder ein oder mehrere Stutzelemente (7) und/oder eine oder mehrere druckempfindliche Haftschichten (13) und/oder eine ablösbare Schutzfolie (12) aufweist.

11. Transdermales Applikationssystem (100) umfassend:
• eine Matrix (3), die zumindest einen sauren Wirkstoff (4) enthält,
• eine erste Elektrode (1), und
• eine zweite Elektrode (2), die für den zumindest einen Wirkstoff (4) durchlässig ausgebildet ist,
wobei die Matrix (3) zwischen der ersten Elektrode (1) und der zweiten Elektrode (2) angeordnet ist und einen pH-Wert aufweist, der größer als der pKs-Wert des zumindest einen Wirkstoffes (4) ist, und wobei das System so ausgelegt ist, dass durch eine an die Elektroden angelegte Spannung der pH-Wert der Matrix (3) nicht unter den pKs-Wert des zumindest einen Wirkstoffes (4) abfällt, wobei der saure Wirkstoff zu mindestens 75 % in deprotonierter Form vorliegt.

12. Transdermales Applikationssystem (100) nach einem der vorhergehenden Ansprüche zur Anwendung in einem Verfahren zur Abgabe eines Wirkstoffes (4), wobei zwischen der ersten Elektrode (1) und der zweiten Elektrode (2) ein oder mehrmals eine Spannung in der Weise angelegt wird, dass im Falle eines basischen Wirkstoffes (4) der pH-Wert der Matrix (3) nicht über den pKs-Wert des zumindest einen Wirkstoffes (4) steigt und im Falle eines sauren Wirkstoffes (4) der pH-Wert der Matrix (3) nicht unter den pKs-Wert des zumindest einen Wirkstoffes (4) fällt.

13. Transdermales Applikationssystem (100) nach einem der vorhergehenden Ansprüche zur Anwendung zur Verabreichung zumindest eines Wirkstoffes durch die Haut.

## Claims

1. A transdermal application system (100) comprising:
• a matrix (3), which contains at least one alkaline active agent (4),
• a first electrode (1), and
• a second electrode (2), which is formed so as to be penetrable for the at least one active agent (4),
wherein the matrix (3) is arranged between the first electrode (1) and the second electrode (2) and has a pH value which is lower than the pKs value of the at least one active agent (4), and wherein the system is designed so that through a voltage applied to the electrodes the pH value of the matrix (3) does not exceed the pKs value of the at least one active agent (4), wherein the alkaline active agent (4) is present at at least 75 % in protonated form.

2. The transdermal application system (100) according to Claim 1, wherein the alkaline active agent (4) is present at at least 90 % and advantageously at at least 99 % in protonated form.

3. The transdermal application system (100) according to Claim 1 or 2, wherein the at least one alkaline active agent (4) is selected from analgesics, µ-opiod-receptor-antagonists, anaesthetising active agents, parasympathomimetics, parasympatholytics, antiemetics, emetics, sympathomimetics, hormones, anti-migraine agents, antiallergics, anticonvulsants, anti-dementia drugs, antidepressants, betablockers and analeptics and mixtures thereof.

4. The transdermal application system (100) according to Claim 3, wherein the at least one alkaline active agent (4) is selected from fentanyl, buprenorphine, physostigmine, rivastigmine, scopolamine, granisetron, zolmitriptan, sumatriptan, salbutamol or mixtures thereof.

5. The transdermal application system (100) according to one of the preceding claims, wherein the matrix (3) contains one or more additives which are selected from penetration-enhancing and/or permeation-enhancing substances, preservatives, antioxidants, humectants, electrolytes, thickening agents, emulsifiers, tackifiers, plasticizers, activation agents, alkalifying agents, buffers and fillers and mixtures thereof.

6. The transdermal application system (100) according to one of the preceding claims, wherein at least the electrode (1) operated as an anode is a silver anode and chloride (Cl⁻) is contained in the matrix (3).

7. The transdermal application system (100) according to one of the preceding claims, which has in addition an adhesive layer (5), which is provided to come into contact with the skin upon application of the system.

8. The transdermal application system (100) according to Claim 7, wherein also the at least one alkaline active agent (4) is contained in the adhesive layer (5).

9. The transdermal application system (100) according to one of the preceding claims, which comprises in addition a covering foil (9) which is impermeable to the active agent and which is preferably gas-permeable.

10. The transdermal application system (100) according to one of the preceding claims, which has in addition a voltage supply, which preferably comprises a voltage source (10) and a control device (11), and/or has one or more spacers (6) and/or one or more support elements (7) and/or one or more pressure-sensitive adhesive layers (13) and/or a detachable protective foil (12).

11. A transdermal application system (100) comprising:
• a matrix (3), which contains at least one acidic active agent (4),
• a first electrode (1), and
• a second electrode (2), which is formed so as to be penetrable for the at least one active agent (4),
wherein the matrix (3) is arranged between the first electrode (1) and the second electrode (2) and has a pH value which is greater than the pKs value of the at least one active agent (4), and wherein the system is designed so that through a voltage applied to the electrodes the pH value of the matrix (3) does not fall below the pKs value of the at least one active agent (4), wherein the acidic active agent is present at at least 75 % in de-protonated form.

12. The transdermal application system (100) according to one of the preceding claims for use in a method for the administering of an active agent (4), wherein a voltage is applied one or more times between the first electrode (1) and the second electrode (2) such that in the case of an alkaline active agent (4) the pH value of the matrix (3) does not increase above the pKs value of the at least one active agent (4), and in the case of an acidic active agent (4) the pH value of the matrix (3) does not fall below the pKs value of the at least one active agent (4).

13. The transdermal application system (100) according to one of the preceding claims for use for the administration of at least one active agent through the skin.

## Revendications

1. Système d'application transdermique (100) comprenant :
• une matrice (3) qui contient au moins un agent actif alcalin (4),
• une première électrode (1), et
• une seconde électrode (2) à travers laquelle l'au moins un agent actif (4) peut passer,
la matrice (3) étant disposée entre la première électrode (1) et la seconde électrode (2) et présentant un taux de pH qui est inférieur au taux de pKs de l'au moins un agent actif (4), et le système étant conçu pour que, suite à une tension appliquée aux électrodes, le taux de pH de la matrice (3) n'augmente pas au-delà du taux de pKs de l'au moins un agent actif (4) le agent actif alcalin (4) étant présent pour au moins 75 % sous forme protonisée .

2. Système d'application transdermique (100) selon la revendication 1, dans lequel le agent actif alcalin (4) est présent pour au moins 90 % et avantageusement pour au moins 99 % sous forme protonisée.

3. Système d'application transdermique (100) selon la revendication 1 ou 2, dans lequel l'au moins un agent actif alcalin (4) est sélectionné parmi les analgésiques, les antagonistes récepteurs de µ-opioïdes, les agents actifs anesthésiants, les parasympathomimétiques, les antiémétiques, les émétiques, les sympathomimétiques, les hormones, les produits anti-migraine, les anti-allergéniques les anticonvulsifs, les antidémentiels, les antidépresseurs, les bêtabloquants et les analeptiques et leurs mélanges.

4. Système d'application transdermique (100) selon la revendication 3, dans lequel l'au moins un agent actif alcalin (4) est sélectionné parmi le fentanyl, la buprénorphine, la physostigmine, la rivastigmine, la scopolamine, le granisétron, le zolmitriptane, le sumatriptane, le salbutamol ou leurs mélanges.

5. Système d'application transdermique (100) selon une des revendications précédentes, dans lequel la matrice (3) contient un ou plusieurs additifs qui sont sélectionnés parmi les substances favorisant la pénétration et/ou la perméation, les conservateurs, les antioxydants, les humectants, les électrolytes, les épaississants, les émulsifiants, les agents d'adhérence, les adoucisseurs, les activeurs, les agendas d'alcalinisation, les tampons et les matières de charge et leurs mélanges.

6. Système d'application transdermique (100) selon une des revendications précédentes, dans lequel au moins l'électrode faisant office d'anode (1) est une anode en argent et la matrice (3) contient du chlorure (Cl⁻).

7. Système d'application transdermique (100) selon une des revendications précédentes, qui présente en outre une couche adhésive (5) qui est prévue pour venir en contact avec la peau lors de l'application du système.

8. Système d'application transdermique (100) selon la revendication 7, dans lequel la couche adhésive (5) contient également l'au moins un agent actif (4).

9. Système d'application transdermique (100) selon une des revendications précédentes, qui comprend en outre un film de couverture perméable aux agents actifs (9) et qui est de préférence conçu pour laisser passer le gaz.

10. Système d'application transdermique (100) selon une des revendications précédentes, qui comprend en outre une alimentation en tension qui comprend de préférence une source de tension (10) et un dispositif de commande (11), et/ou un ou plusieurs espaceurs (6) et/ou un ou plusieurs éléments d'appui (7) et/ou une plusieurs couches adhésives sensibles à la pression (13) et/ou un film protecteur détachable (12).

11. Système d'application transdermique (100) comprenant :
• une matrice (3) qui contient au moins un agent actif acide (4)
• une première électrode (1), et
• une seconde électrode (2) à travers laquelle l'au moins un agent actif (4) peut passer,
la matrice (3) étant disposée entre la première électrode (1) et la seconde électrode (2) et présentant un taux de pH qui est supérieur au taux de pKs de l'au moins un agent actif (4), et le système étant conçu pour que, suite à une tension appliquée aux électrodes, le taux de pH de la matrice (3) ne diminue pas en dessous du taux de pKs de l'au moins un agent actif (4) le agent actif acide étant présent pour au moins 75 % sous forme déprotonisée .

12. Système d'application transdermique (100) selon une des revendications précédentes, destiné à une utilisation dans un procédé de délivrance d'un agent actif (4), une tension étant appliquée une ou plusieurs fois entre la première électrode (1) et la seconde électrode (2) de manière à ce que, dans le cas d'un agent actif alcalin (4), le taux de pH de la matrice (3) n'augmente pas au-delà du taux de pKs de l'au moins un agent actif (4) et, dans le cas d'un agent actif acide (4), le taux de pH de la matrice (3) ne diminue pas en dessous du taux de pKs de l'au moins un agent actif (4).

13. Système d'application transdermique (100) selon une des revendications précédentes, destiné à une utilisation pour administrer au moins un agent actif à travers la peau.
